# EUROPEAN PATENT APPLICATION

(11) **EP 2 913 033 A1**
(43) Date of publication of application: **02.09.2015**
(21) Application number: 13850983.1
(22) Date of filing: 21.10.2013
(51) Int. Cl.: A61F 2/95

(54) **STENT GRAFT INDWELLING DEVICE**

(30) Priority: 29.10.2012 JP 2012237361
(71) Applicant: Kawasumi Laboratories, Inc., Oita 876-0121 (JP)
(72) Inventor: AKITA, Kazumi, Bungo-Ono-Shi Oita 879-7153 (JP); SEKI, Kengo, Bungo-Ono-shi Oita 879-7153 (JP)
(74) Representative: Hering, Hartmut
(86) International application number: PCT/JP2013/078491
(87) International publication number: WO 2014/069275

(57) **Abstract**

In a stent graft indwelling device including a sheath 2, a sheath hub 3, a dilator 5, and anfront tip 7, "pressure resistance performance" of the sheath hub and "fixing performance" of the dilator are steadily improved. A lock cap 3.1 and a hemostatic valve 3.5 are arranged as pressure resistant members at the proximal end PE side of the sheath hub 3, the lock cap 3.1 having a large-diameter portion 3.1LD, a small-diameter portion 3.1SD, and a fin portion 3.1FN and being fitted to the proximal end PE side of the sheath hub 3. The large-diameter portion 3.1LD is formed, on its interior at the proximal end PE side, with an undercut portion 3.1UC having an annular step and the small-diameter portion 3.1SD is fitted, at the proximal end PE side, with the fin portion 3.1FN. The hemostatic valve 3.5 has a substantially planar plate shape and has a flange 3.5F, the flange 3.5F of the hemostatic valve 3.5 being fitted to the annular step of the undercut portion 3.1UC of the lock cap 3.1.

## Description

### TECHNICAL FIELD

The present invention relates to a stent graft indwelling device (hereinafter, may be referred to simply as "indwelling device") that, for the purpose of treating, e.g., arterial abnormal distention disease (aneurysm, etc.) and artery stenosis or other diseases, places or indwells a stent graft for use in treatment of the aneurysm, etc., at a safe portion in a diseased site.

More particularly, the present invention relates to a stent graft indwelling device having a sheath, a sheath hub, a dilator, and a front tip, in which a stent graft is transported through a blood vessel, while being hooked to the front tip, to a site where an aneurysm is developed, etc. , and is indwelled there. The stent graft indwelling device has remarkably improved performances or capabilities of members (hereinafter, each may be referred to as a "pressure resistant member", a "fixing member" , and an "air bleeding member") which are related respectively to a "pressure resistance performance" (also called a "blood backflow prevention performance") of the sheath hub, a "fixing performance" of the dilator, and an "air bleeding performance" in priming to the sheath and the sheath hub.

A main member acting as the "pressure resistant member" of the present invention is for example a lock cap (also called "valved hub cap") or a hemostatic valve fitted to the sheath hub at the proximal end side; a main member acting as the "fixing member" is for example a lock cap or nut; and a main member acting as the "air bleeding member" is for example a rectifying member.

### BACKGROUND ART

The applicant of this invention has previously disclosed basic inventions relating to the stent graft indwelling device in Patent Documents 1 (International Publication WO2005/99806) and 2 (International Publication WO2008/143243). The objects of the present invention are to remarkably improve the pressure resistance performance, the fixing performance, and the air bleeding performance, while maintaining or preserving the superior characteristics of the basic indwelling devices, thereby further enhancing the degree of completeness of the device as a whole.

As to the pressure resistance performance and the fixing performance, in the inventions described in Patent Documents 1 and 2 (hereinafter, may be referred to as "Patent Document 1, etc."), the valved hub cap (see reference numeral 37 of Fig. 1 of the documents; The same shall apply hereafter) is secured to the sheath hub at the proximal end side so that arterial blood does not leak when the dilator is inserted into the sheath.

That is, the valved hub cap has the hemostatic valve fitted in the interior thereof and acts to maintain the pressure resistance performance against the pressure of blood flow in the artery and to immovably fix the dilator after the insertion of the dilator into the sheath.

The valved hub cap consists of two pieces (a cap body and a cap), with a valve interposed between the cap body and the cap which are heat welded together for assembly. The cap body has at its end a fitting portion (or threaded portion) that is fitted to (or screwed with) the sheath hub at the proximal end side.

Further, as to the air bleeding performance, in the inventions of Patent Document 1, etc., a priming liquid (physiological saline solution or distilled water) is introduced into the sheath (see reference numeral 30) through one liquid filling port (see reference numeral 32) formed on one side in the vicinity of the valved hub cap (see reference numeral 37).

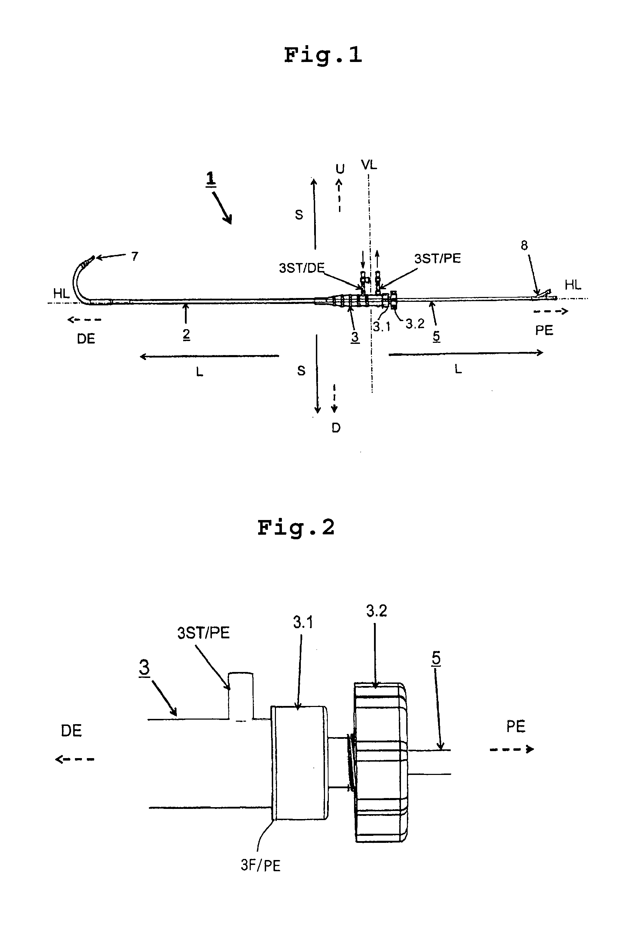

The priming liquid is introduced into the sheath through a gap (space) between the dilator and the sheath, whereas air generated in the sheath or remaining in a void is discharged through a concaved fluid passage (see reference numeral 17 in Fig. 2 of the Documents) extending longitudinally along the side of the dilator and then purged outwardly from the other liquid filling port (see reference numeral 32).

### PRIOR ART DOCUMENT

### PATENT DOCUMENT

Patent Document 1: International Publication WO2005/99806 (see Figs. 1 and 2 and pp. 6-7)
Patent Document 2: International Publication WO2008/143243 (see Figs. 1, 2, and 4 and paragraphs [0048]-[0068])

### SUMMARY OF THE INVENTION

### PROBLEMS TO BE SOLVED BY THE INVENTION

Although the stent graft indwelling devices of the inventions described in Patent Document 1, etc., have the basic pressure resistance performance or capability and fixing performance, they suffer from the following problems due to the manner in which the valved hub cap is assembled by heat-welding process prior to securing to the sheath hub.
(a) Even a slight deformation in the shape of the welded part after heat-welding may cause instability in the pressure resistance and fixing performance.
(b) Involvement of heat welding instruments for welding increases the number of manufacturing processes.
(c) Referring to the air bleeding performance, the sheath hub (see reference numeral 31) is formed with a size of relatively large diameter and large volume compared among the constitution members of the indwelling device. It has therefore been found that air generated in the sheath and the sheath hub during the priming tends to stay in the sheath hub at the proximal end side and thus a rapid air bleeding is difficult to perform through the concaved fluid passage extending longitudinally along the side of the dilator.

### MEANS FOR SOLVING PROBLEMS

As a result of intensive studies to solve the problems, the inventors have conceived an invention with respect to an novel stent graft indwelling device including a lock cap and a hemostatic valve, etc. , as a novel "pressure resistant member" ; a lock cap and a nut, etc., as a novel "fixing member"; and a rectifying member, etc., as a novel "air bleeding member" , which succeeded in remarkably improving its pressure resistance performance, fixing performance, and air bleeding capability and thus further enhancing the degree of completeness of the device as a whole. The present invention is as follows.
[1] The present invention provides a stent graft indwelling device (1) comprising a sheath (2), a sheath hub (3), a dilator (5), and a front tip (7),
   the front tip (7), the sheath (2), and the sheath hub (3) being disposed from a distal end DE toward a proximal end PE in this order,
   the dilator (5) being inserted from the proximal end PE side of the sheath hub (3) toward the distal end DE side of the sheath (2),
   a lock cap (3.1) and a hemostatic valve (3.5) being arranged as pressure resistant members at the proximal end PE side of the sheath hub (3),
   the lock cap (3.1) having a large-diameter portion (3.1LD), a small-diameter portion (3.1SD), and a fin portion (3.1FN),
   the large-diameter portion (3 .1LD) having at the proximal end PE side a large-diameter portion wall (3.1LDW),
   the large-diameter portion (3.1LD) having on its interior at the proximal end PE side an undercut portion (3.1UC) comprised of an annular step portion,
   the hemostatic valve (3.5) having generally a form of a planar plate, and having at the proximal end PE side a flange (3.5F) and having at its substantially center an insertion opening (3.50), wherein
   the flange (3.5F) of the hemostatic valve (3.5) is mounted to the annular step portion of the undercut portion (3.1UC) of the lock cap (3.1),
   thereby fixing the hemostatic valve (3.5) to the undercut portion (3.1UC),
   the large-diameter portion (3.1LD) having a large-diameter portion fitting portion (3.1LDSN) formed on its interior at the distal end DE side,
   the distal end DE side of the small-diameter portion (3.1SD) being connected to a substantial center of the large-diameter portion wall (3.1LDW),
   the small-diameter portion (3.1SD) having a small-diameter portion fitting portion (3.1SDSN) formed on its exterior at the proximal end PE side,
   the small-diameter portion (3.1SD) being fitted, at the proximal end PE side, with the fin portion (3.1FN),
   the fin portion (3.1FN) having a plurality of fins (3.1FF),
   the large-diameter portion (3.1LD) at the distal DE side of the lock cap (3.1) being fitted to the proximal end PE side of the sheath hub (3).
[2] The present invention also provides the stent graft indwelling device (1) of [1], wherein
   the lock cap (3.1) defined in [1] and a nut (3.2) are arranged at the proximal end PE side of the sheath hub (3), as fixing members for the dilator (5),
   the nut (3.2) having an outer housing portion (3.2HO) and an inner-tube portion (3.21),
   the outer housing portion (3.2HO) having at the proximal end PE side a wall (3.2W), and the inner-tube portion (3.21) projecting from a substantial center of the wall (3.2W) toward the distal end DE side,
   the inner-tube portion (3.2I) having an inner-tube portion fitting portion (3.2ISN) formed on its interior at the distal end DE side,
   the inner-tube portion (3.2I) having an inner-tube portion projecting portion (3.2IT) formed on its interior at the proximal end PE side,
   the nut (3.2) having an insertion opening (3.20) substantially at its center, and wherein
   the nut (3.2) is rotationally displaced toward the distal end DE direction, thereby
   external tightening accompanied by the displacement of the nut (3.2) causes the plurality of fins (3.1FF) of the lock cap (3.1) to close toward the center so that the dilator (5) can be fixed immovably.
[3] The present invention also provides a stent graft indwelling device (1) comprising a sheath (2), a sheath hub (3), a dilator (5), and a front tip (7),
   the front tip (7), the sheath (2), and the sheath hub (3) being disposed from a distal end DE toward a proximal end PE in this order,
   the dilator (5) being inserted from the proximal end PE side of the sheath hub (3) toward the distal end DE side of the sheath (2),
   the sheath hub (3) having on its exterior a distal-end-side hub side-tube (3ST/DE) and a proximal-end-side hub side-tube (3ST/PE),
   a rectifying member (3.3) being disposed as an air bleeding member on the interior at the proximal end PE side of the sheath hub (3),
   the rectifying member (3.3) having a substantially tubular shape and having at the distal end DE side an inclined wall (3.3W),
   a top portion (3.3WT) of the inclined wall (3.3W) being positioned in the vicinity of an opening (3STO) of the proximal-end-side hub side-tube (3ST/PE), wherein
   air generated in a priming liquid introduced from the distal-end-side hub side-tube (3ST/DE) into the sheath hub (3) and into the sheath (2) can be discharged outwardly through a passage or by way of the inclined wall (3.3W) of the rectifying member (3.3), the top portion (3.3WT), and the opening (3STO) of the proximal-end-side hub side-tube (3ST/PE).
[4] The present invention also provides the stent graft indwelling device (1) of [3], wherein
   an angle of the inclined wall (3.3W) of the rectifying member (3.3) is formed in the range of 30° to 60°.
[5] The present invention also provides the stent graft indwelling device (1) of any one of [1] to [4], wherein
   the exterior of a connection between the proximal end PE side of the sheath (2) and the distal end DE side of the sheath hub (3) is covered with a strain relief (3SR),
   the strain relief (3SR) having a substantially tubular shape and being formed, with a fit-in groove (3SRM) on its interior at the proximal end PE side,
   a distal-end-side hub flange (3F/DE) of the sheath hub (3) being fitted in the fit-in groove (3SRM).
[6] The present invention also provides the stent graft indwelling device (1) of any one of [1] to [5], wherein
   the exterior of the sheath hub (3) is covered with a hub grip (3GP),
   the hub grip (3GP) having along its longitudinal L direction a plurality of connection tubes (3GPCT) and a plurality of connecting members (3GPS),
   the plurality of connection tubes (3GPCT) being connected to each other so as to form a gap therebetween or apart from each other with a space therebetween,
   the connection tube (3GPCT) being formed, on its exterior, with an uneven portion (3GPCTK).

### ADVANTAGEOUS EFFECT OF THE INVENTION

(1) According to the stent graft indwelling device of the present invention, combination of, or cooperation of the two members, the lock cap 3.1 and the hemostatic valve 3.5 defined in the present invention enables the sheath hub to have a steadily and remarkably improved "pressure resistance performance" (also called "blood backflow prevention capability").
(2) And according to the stent graft indwelling device of the present invention, combination of, or cooperation of the two members, the lock cap 3.1 and the nut 3.2 defined in the present invention enables the dilator to have a steadily and remarkably improved "fixing performance", whereby the stent graft which is fitted or mounted to the sheath 2 at the distal end DE side of the dilator 5 can be protected and prevented from being pushed out of the sheath.
(3) Further, according to the stent graft indwelling device of the present invention, introduction of the rectifying member 3.3 defined in the present invention achieves a remarkable improvement in the "air bleeding capability" during priming procedure of the sheath 2 and the sheath hub 3, whereupon air in the sheath 2 generated during the priming can promptly be discharged outwardly facilitated by the presence of an inclined wall 3.3W in particular.

### BRIEF DESCRIPTION OF DRAWINGS

[Fig. 1] Fig. 1 is a general side view of a stent graft indwelling device of the present invention, viewed from the lateral (first lateral S1) direction.
[Fig. 2] Fig. 2 is a partially enlarged view of the vicinities of a lock cap 3.1 and a nut 3.2 at the proximal end PE side of the sheath hub 3, viewed from the lateral (first lateral S1) direction.
[Fig. 3] Fig. 3 is a longitudinal sectional view along the longitudinal L direction of Fig. 2.
[Fig. 4] Fig. 4 is a perspective view of the nut 3.2 of Fig. 3, viewed from the lateral (first lateral S1) direction and from the proximal end PE direction.
[Fig. 5] Fig. 5 is a side view of the lock cap 3.1 of Fig. 4, viewed from the proximal end PE direction.
[Fig. 6] Fig. 6 is a longitudinal sectional view, along the longitudinal L direction, of the lock cap 3.1, the nut 3.2, and their vicinities of Fig. 3, depicting the state thereof in operation.
[Fig. 7] Fig. 7 is a partially enlarged sectional view of a hemostatic valve 3.5 in its vicinities shown in Fig. 6.
[Fig. 8] Fig. 8 is a partially enlarged sectional view of the hemostatic valve 3.5 in its vicinities shown in Fig. 7, depicting the state thereof in operation.
[Fig. 9] Fig. 9(A) is a perspective view of the lock cap 3.1, viewed from the lateral (first lateral S1) direction and from the proximal end PE direction; and Fig. 9(B) is a longitudinal sectional view along the longitudinal L direction of (A).
[Fig. 10] Fig. 10 is a schematic view of the stent graft indwelling device in the state in operation during the priming.
[Fig. 11] Fig. 11 is a partially enlarged sectional view of a rectifying member 3.3 in its vicinities shown in Fig. 3.
[Fig. 12] Fig. 12(A) is a perspective view of the nut 3.2, viewed from the lateral (first lateral S1) direction and from the proximal end PE direction; Fig.12(B) is a side view of the nut 3.2 viewed from the proximal end PE direction; Fig. 12(C) is a longitudinal sectional view of the nut 3.2, viewed from the longitudinal L direction; and Fig. 12(D) is a partially enlarged view of an inner-tube portion fitting portion 3.2ISN in its vicinities (area B) shown in Fig. 12(C).
[Fig. 13] Fig. 13(A) is a side view of the hub 3, viewed from the upper portion U direction; Fig. 13(B) is a partially cut-away sectional view, along the longitudinal L direction, of the hub 3, viewed from the lateral (first lateral S1) direction; Fig. 13(C) is an enlarged view of a part A of Fig. 13(B) ; Fig. 13(D) is a side view of Fig. 13(A), viewed from the proximal end PE; and Fig. 13(E) is a sectional view of Fig. 13(B) taken along line B-O-B'.
[Fig. 14] Fig. 14(A) is a partially cut-away sectional view, along the longitudinal L direction, of a strain relief; Fig. 14(B) is a side view of Fig. 14(A), viewed from the proximal end PE; and Fig. 14(C) is a partially enlarged view (detailed view) of an area A' of Fig. 14(A).
[Fig. 15] Fig. 15 (A) is a side view of a nut weight 3.2WG, viewed from the proximal end PE direction; and Fig. 15 (B) is a partially cut-away sectional view, along the longitudinal L direction, of the nut weight 3.2WG.
[Fig. 16] Fig. 16(A) is a plan view of a hub grip 3GP, viewed from the upper portion U direction; Fig. 16(B) is a partially cut-away sectional view of the hub grip 3GP, along the longitudinal L direction, when viewed from the lateral (first lateral S1) direction; Fig. 16(C) is a sectional view of Fig. 16(B) taken along line A-A'; Fig. 16(D) is a sectional view of Fig. 16 (B) taken along line B-B'; and Fig. 16 (E) is a sectional view of Fig. 16(A) taken along line E-E'.
[Fig. 17] Fig. 17(A) is a partially cut-away sectional view of the lock cap 3.1, along the longitudinal L direction, when viewed from the lateral (first lateral S1) direction; and Fig. 17(B) is a side view of Fig. 17(A), viewed from the proximal end PE direction.
[Fig. 18] Fig. 18 (A) is a plan view of the hemostatic valve 3.5, viewed from the proximal end PE direction; and Fig. 18(B) is a partially cut-away sectional view of a side surface, along the longitudinal L direction, of Fig. 18(A).
[Fig. 19] In Figs. 19(A) to (D), Fig. 19(A) is a longitudinal sectional view, along the longitudinal L direction, of the rectifying member 3.3, when viewed from the lateral (first lateral S1) direction; Fig. 19 (B) is a side view of Fig. 19(A), viewed from the proximal end PE direction; Fig. 19 (C) is a side view of Fig. 19(A), viewed from the distal end DE direction; and Fig. 19(D) is a sectional view of Fig. 19(B) taken along line A-A'.
[Fig. 20] Fig. 20(A) is a perspective view of the front tip 7, viewed from the lateral (first lateral S1) direction, the upper portion U direction and the proximal end PE direction; and Fig. 20 (B) is a perspective view from the lateral (first lateral S1) direction and the upper portion U direction.
[Fig. 21] Fig. 21 (A) is a schematic view of the front tip 7 whose proximal end PE side is covered with the distal end DE side of the sheath 2; and Fig. 21(B) is a partially enlarged view of Fig. 21(A).
[Fig. 22] Fig. 22 is a side view of the sheath 2, viewed from the lateral (first lateral S1) direction.

### MODES FOR CARRYING OUT THE INVENTION

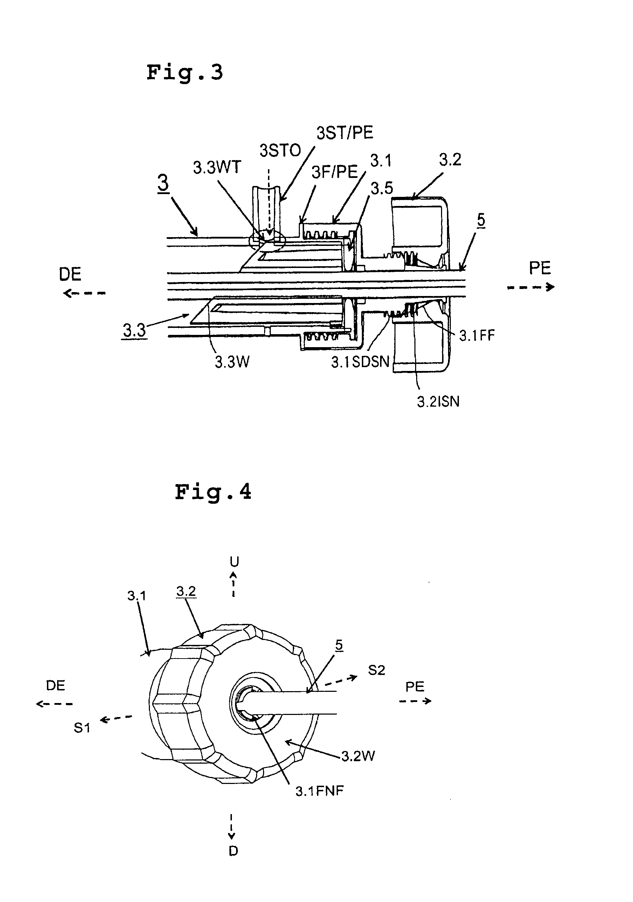

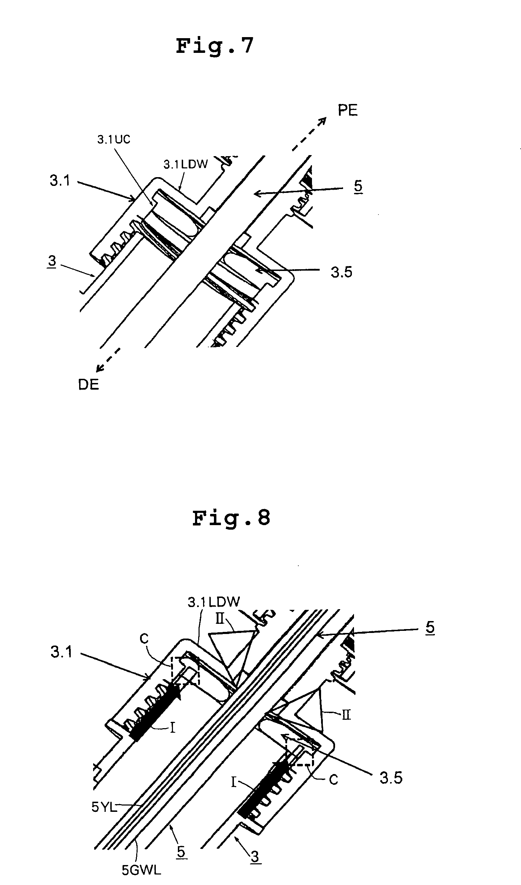

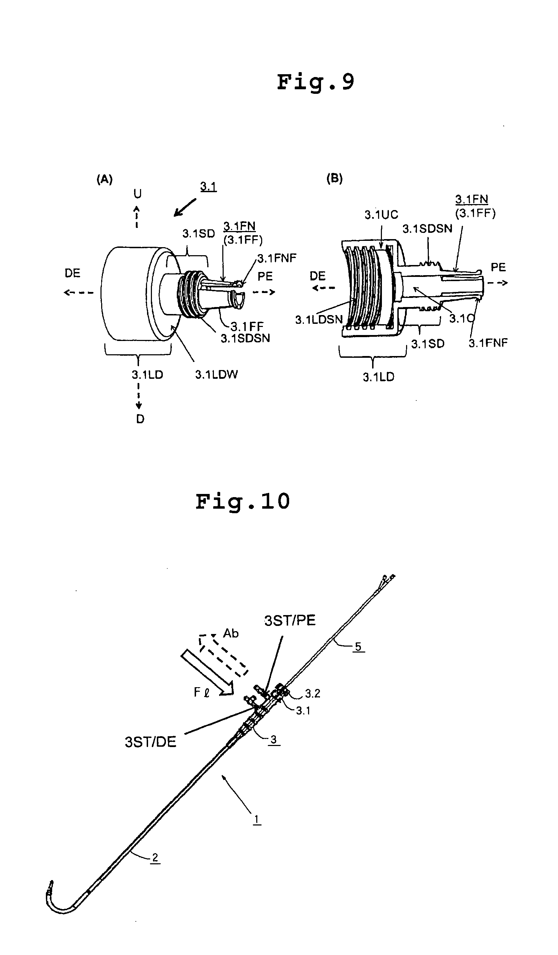

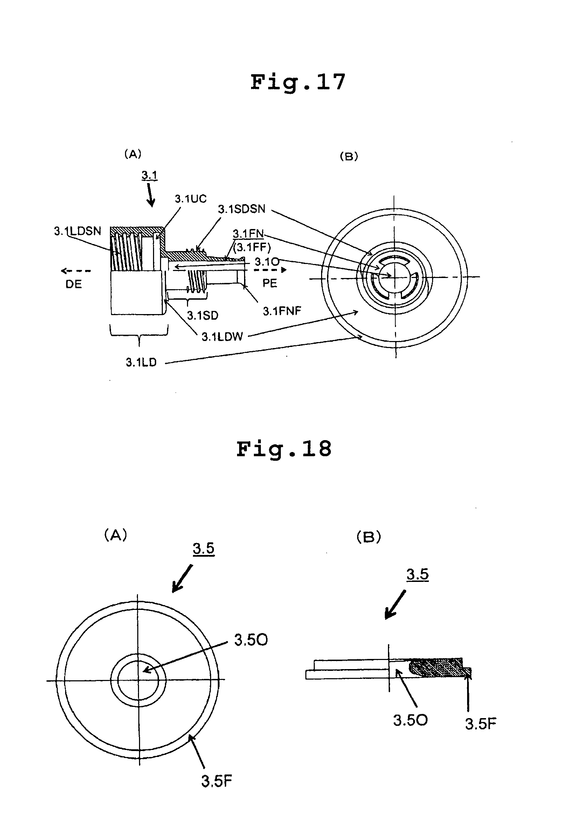

The present invention will now be described in detail with reference to the drawings. In order to more clearly describe the present invention, the following definitions will be given based on descriptions of the drawings.
(Definition 1) In a stent graft indwelling device of the present invention, "first state" means the state where a large-diameter portion 3. 1LD (see Fig. 9) of a lock cap 3.1 closer to a distal end DE is fitted to a sheath hub 3 at a proximal end PE side, as exemplified in Fig. 7.
(Definition 2) "Second state" means the state where a dilator 5 is secured into a sheath hub 3, with the interior (inner peripheral portion) of a hemostatic valve 3.5 shaped as in Fig. 18 being in intimate contact with the exterior of the dilator 5, as depicted in Fig. 8.
(Definition 3) "Transition state" means the intermediate state when shifting from the "first state" to the "second state" is made.
(Definition 4) "Transition start state" means the initial state when shifting from the "first state" to the "second state" is made.
(Definition 5) "Transition end state" means the final state immediately before the end of the shifting from the "first state" to the "second state".
(Definition 6) In the present invention, "proximal end PE (side or direction)" means the end portion on a side fitted with a proximal end connector 8, as exemplified in Fig. 1.
(Definition 7) "Distal end DE (side or direction)" means the end portion opposite to the "proximal end PE (side or direction)", as exemplified in Fig. 1. Furthermore, it means an end portion (side or direction) of a sheath 2 on a side fitted with anfront tip 7.
(Definition 8) "Horizontal axis HL (direction)" means the longitudinal direction (see a broken line of Fig. 1) along which the stent graft indwelling device extends, as exemplified in Fig. 1.
(Definition 9) "Vertical axis VL (direction)" means the direction (see a broken line of Fig. 1) substantially orthogonal to the longitudinal direction and extends in this direction, as exemplified in Fig. 1.
(Definition 10) "First lateral S1 (side or direction)" means for example the direction (see a broken line arrow of Fig. 4) toward a left-hand end portion of a nut 3.2, as exemplified in Fig. 4. In the exemplification of Fig. 1, it means a front direction of the stent graft indwelling device.
(Definition 11) "Second lateral S2 (side or direction)" means for example the direction (see a broken line arrow of Fig. 4) toward a right-hand end portion of the nut 3.2, as exemplified in Fig. 4. In the exemplification of Fig.1, it means a rear direction of the stent graft indwelling device.
(Definition 12) "Upper portion U (side or direction) " means for example the direction (see a broken line arrow of Fig. 1) in which a hub side-tube 3ST is fitted to the sheath hub 3, as exemplified in Fig. 1.
(Definition 13) "Lower portion D (side or direction) " means for example the opposite side (see a broken line arrow of Fig. 1) to the direction in which the hub side-tube 3ST is fitted, as exemplified in Fig. 1.
(Definition 14) "Longitudinal L direction" means the longitudinal direction (horizontal axis HL (direction)) of the entire stent graft indwelling device, as exemplified in Fig. 1.
(Definition 15) Simply "portion S (side or direction)" means all the directions among the "first lateral S1 (side or direction)", the "second lateral S2 (side or direction)", the "upper portion U (side or direction)" and the "lower portion D (side or direction)".
(Definition 16) "Interior" contains the meanings of "inner periphery, inner wall portion, and lateral S direction" in the case of the "large-diameter portion 3.1 LD" of the lock cap 3.1 for example.
(Definition 17) "Exterior" contains the meanings of "outer periphery, outer wall portion, and lateral S direction" in the case of the "hub 3" for example.
(Definition 18) "○○ fitting portion (reference letter and numeral○○SN)" contains the meaning of "threaded portion or screw portion" in the case of a "large-diameter portion fitting portion 3.1LDSN" of the lock cap 3.1 for example.
(Definition 19): (About descriptions of reference letters and numerals indicative of directionality such as position and arrangement in designations of components)

In the drawings of the present invention and in the description of the present invention, the hub side-tubes 3ST are expressed for exampleas a proximal-end-side hub side-tube with a reference letter and numeral "3ST/PE", as exemplified in Fig.3. In designations of components including the hub side-tubes 3ST, reference letters and numerals indicative of directionality such as position and arrangement, such as "proximal-end PE side" and "first lateral S1 side" are imparted to only some of the components in the drawings and description of the present invention, as long as they are considered necessary and contributing to the understanding of the present invention.

### [Stent Graft Indwelling Device 1]

A stent graft indwelling device 1 (hereinafter, may be referred to simply as "indwelling device 1") of the present invention will now be described with reference to the drawings (Figs. 1 to 22).

The stent graft indwelling device 1 of the present invention includes, as exemplified in Fig. 1, the sheath 2, the sheath hub 3 holding the sheath, the dilator 5, the front tip 7, and the proximal end connector 8.

The stent graft indwelling device 1 is configured or disposed such that the front tip 7, the sheath 2, the sheath hub 3, and the proximal end connector 8 are arranged in this order from the distal end DE toward the proximal end PE.

As depicted in Fig. 1, the dilator 5 is fitted or mounted with the proximal end connector 8 at its proximal end PE side, and is fitted or mounted, at its distal end DE side, with the front tip 7 at the proximal end PE side.

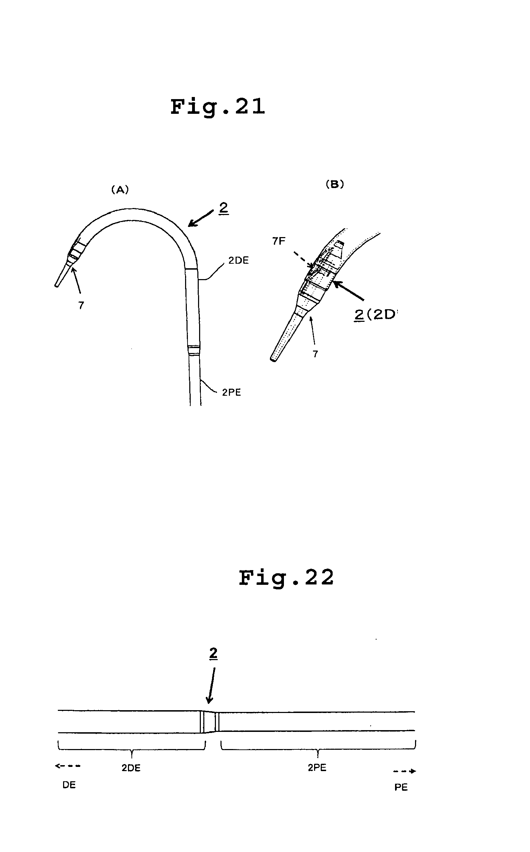

More specifically, the dilator 5 is loaded (inserted) from the proximal end PE side of the sheath hub 3 toward the distal end DE side of the sheath 2, with the front tip 7 being fitted as depicted in Fig. 21. The structure, etc., of the dilator 5 will be described later.

Forms and functions of these indwelling device constituting members are as follows.

### [Sheath 2]

The sheath 2 is formed from a tube having an inner diameter size large enough to accommodate the dilator 5 and a stent graft (dare not be designated by reference letter and numeral; see reference numeral 60 of Patent Document 2), as exemplified in Figs. 1 and 22.

The sheath 2 has a more reduced diameter at the proximal end PE than at the distal end DE which holds the front tip, as exemplified in Fig. 22, so that the passing resistance of the sheath can be reduced when moving through a blood vessel.

The diameter of the sheath 2DE on the distal end DE side can be set to various specifications such as 21, 22, and 23Fr for example, taking into consideration the specifications of the stent graft to be loaded and the blood vessel diameter of an access route.

On the other hand, the diameter of the sheath 2PE on the proximal end PE side can be set, as a whole, to a common value (or a certain, constant diameter) within the above mentioned limits on the specification of diameter.

The sheath 2 is made of a material having a flexibility. Although the flexible material is not specifically limited, it could be, for example, a simple synthetic resin such as a fluorocarbon resin, a polyamide resin, a polyamide-based elastomer, and a polyvinyl chloride resin; a compound of these resins; a multi-layered structure of these resins; or a composite of these resins and metal wire, etc., (the same applies to a strain relief 3SR and a hub grip 3GP which will be described later).

### [Sheath Hub 3]

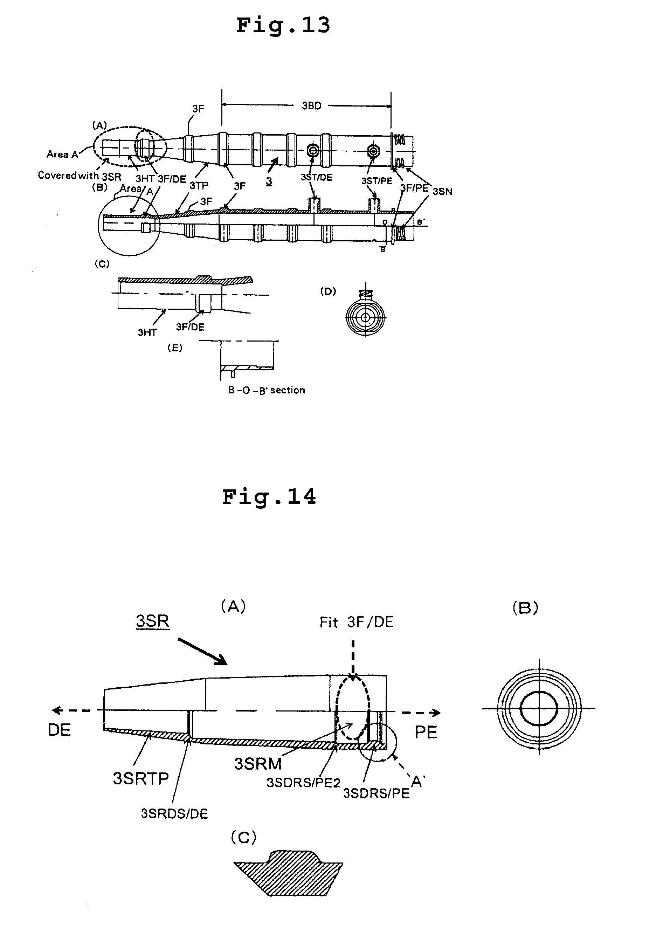

The sheath hub 3 (hereinafter, may be referred to simply as "hub 3") includes a thin or small tube portion 3HT, a tapered portion 3TP, a hub flange 3F, a hub base body 3BD, a hub side-tube 3ST, and a hub fitting or mounting portion 3SN, as exemplified in Figs. 1 to 3, 6, and esp., Fig. 13.

The hub 3 has at its distal end DE side the thin or small tube portion 3HT that extends substantially straightforwardly, and has a distal-end-side hub flange 3F/DE formed on the proximal end PE side of the thin or small tube portion 3HT (see area A of Fig. 13).

The tapered portion 3TP is so configured as to extend to connect with the distal-end-side hub flange 3F/DE at the proximal end PE side.

The tapered portion 3TP is formed so as to descend from the proximal end PE side toward the distal end DE side, i.e., the tapered portion has a gradually reducing diameter toward the distal end DE side.

On the other hand, a plurality of hub flanges 3F are formed on the tapered portion 3TP on the proximal end PE side (two hub flanges are formed on the tapered portion in Fig. 13).

The hub base body 3BD in the form of a tubular body is connected with the tapered portion 3TP and is formed with a plurality ofhub flanges 3F, of which a hub flange 3F/PE, the closest one to the proximal end PE, has, formed on its exterior ("outer periphery", "outer wall portion", or "laterals direction"; the same shall apply hereinafter) on the proximal end PE side, a hub fitting or mounting portion 3SN (may be referred to as "threaded portion" or "screw portion"; the same shall apply hereinafter).

The hub 3 (including the thin or small tube portion, the tapered portion, the hub base body, etc.) is made of a hard material, e.g., polycarbonate resin.

The sheath hub 3 is, more precisely, provided with a set of hub side-tubes 3ST (two hub side-tubes 3ST are disposed at two locations) having an inlet for priming liquid and an outlet for air, on the exterior extending from substantially the vicinity of the middle portion of the hub base body 3BD toward the proximal end PE, as will be described hereinafter.

Hereinafter, for convenience of description, the hub side-tube 3ST closer to the proximal end PE will be referred to as proximal-end-side hub side-tube"3ST/PE". The hub side-tube 3ST closer to the distal end DE (in Fig. 13, positioned substantially in the vicinity of the middle point) will be described as distal-end-side hub side-tube"3ST/DE".

For example, as depicted in Fig. 10, the distal-end-side hub side-tube 3ST/DE is used when a priming liquid (distilled water or physiological saline solution) Fl is injected from the sheath hub 3 into the sheath 2.

The proximal-end-side hub side-tube 3ST/PE is used when bleeding or discharging air Ab generated during priming in the sheath 2 and the sheath hub 3, i. e. the air existing in the sheath 2 and the sheath hub 3 and displaced by the priming liquid to the outside of the device.

In the exemplification of Fig. 1, a three-way stopcock or the like (dare not be designated by reference letter and numeral) is fitted or mounted to the hub side-tube 3ST on the upper U side.

### [Strain Relief 3SR]

### (Function)

In the present invention, a strain relief 3SR is preferably disposed as exemplified in Fig. 14.

As exemplified in the diagrams, the strain relief 3SR is a covering member that covers the exterior of a connecting portion between the proximal end PE side of the sheath 2 and the distal end DE side of the sheath hub 3, and functions as an "anti-kink cover".

When connecting the sheath hub 3 and the sheath 2 on to the exterior of the small or thin tube portion 3HT of the hub 3 at the distal end DE side is fitted or mounted (i.e. , adhered and secured by an adhesive) the interior of the sheath 2 at the proximal end PE side (inner periphery, inner wall portion, or lateral S direction; the same shall apply hereinafter), and the strain relief 3SR thus covering and reinforcing the exterior of the vicinity of the small tube portion 3HT keeps the vicinity of the fitting portion adhered and secured by the adhesive from kinking, namely, from bending.

Even in case the adhesive material is to be exposed to the outside, the strain relief provides a coverage over the otherwise exposed portion and thus keeps the good appearance of the product.

### (Shape)

An example of a preferred shape of the strain relief 3SR will hereinafter be described in detail with reference to Fig. 14.

The strain relief 3SR has schematically a so-called "substantially tubular" shape.

The strain relief 3SR has in its interior at the proximal end PE side a plurality of stepped portions 3SRDS (3SRDS/DE, 3SRDS/PE).

A fit-in groove 3SRM is formed and extends between a stepped portion 3SRDS/PE at the proximal end PE side and a second stepped portion 3SRDS/PE2 when viewed from the proximal end PE. The fit-in groove 3SRM is formed to have dimensions (width, depth T, etc.) allowing the distal-end-side hub flange 3F/DE of the hub 3 (more precisely, the flange connecting the thin or small tube portion and the tapered portion, as depicted in Fig. 13) to be fitted therein.

The distal end DE side of the thin tube portion 3HT of the hub 3 comes into abutment against the distal endDE-side stepped portion 3SRDS/DE. Thus, the area A depicted in Fig. 13 is fitted in the space between 3SRDS/DE and 3SRDS/PE of Fig. 14(A).

The strain relief 3SR has a taper portion 3SRTP toward the distal end DE.

Preferably, the strain relief 3SR is made of a resin material, etc., having flexibility that has been described in the previous column of the sheath 2. The detailed exemplification thereof will be omitted.

### [Hub Grip 3GP]

In the present invention, a hub grip 3GP (a covering member) is preferably mounted on the hub for improving its holding property. That is, more specifically, the hub grip 3GP is the covering member having an anti-slip function that is mounted to cover the exterior of the sheath hub 3.

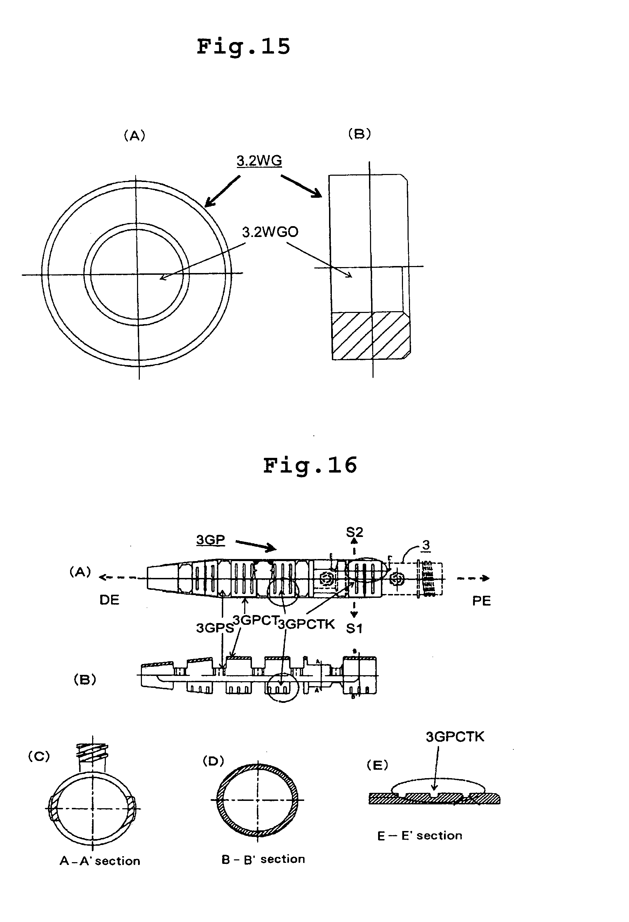

As exemplified in Fig. 16, the hub grip 3GP has a plurality of "substantially cylindrical connection tubes 3GPCT" and a plurality of "connecting members 3GPS" along the longitudinal direction.

The plurality of connection tubes 3GPCT are connected to one another with a predetermined space (dare not be designated by reference letter and numeral) therebetween or connected apart from one another so as to form a gap therebetween.

The exterior (outer surface) of the connection tube 3GPCT has a plurality of uneven portions 3GPCTK.

As described above, the hub grip 3GP is the covering member that is mounted to cover the exterior of the hub 3, with a broken line of Fig. 16 (A) indicating the hub 3 covered with the member.

The user (a doctor or medical staff) grasps the hub grip 3GP when holding the hub 3, to thereby more easily hook the fingers thereon so that the hub 3 can be more firmly held and secured.

In this manner, the hub grip 3GP functions as an anti-slip means for securely holding the hub 3.

The hub grip 3GP is preferably made of a resin material, etc., having flexibility that has been described in the column of the sheath 2. The detailed exemplification thereof will be omitted.

### [Lock Cap 3.1]

As exemplified in Figs. 3, 6 to 9, and 17, the lock cap 3.1 is a member that is fitted with the hemostatic valve 3.5 therein and fastened into the hub 3 at the proximal end PE side, to thereby achieve an improved pressure resistance performance. As will be described later, the lock cap 3.1 has a through-hole 3.10 into which the dilator 5 is inserted and fixed.

As depicted in Figs. 9(A), (B) and Figs. 17(A), (B), the lock cap 3.1 has a large-diameter portion 3.1LD, a small-diameter portion 3.1SD, and a fin portion (or a fin member) 3.1FN.

### (Large-diameter Portion 3.1LD)

The large-diameter portion 3.1LD has a so-called "substantially cylindrical" shape.

The large-diameter portion 3.1LD has an opening (dare not be designated by reference letter and numeral) at the distal end DE side and has a large-diameter portion wall 3.1LDW at the proximal end PE side.

The small-diameter portion 3.1SD at its distal end DE side is connected to the large-diameter portion wall 3.1LDW at its substantially middle point (dare not be designated by reference letter and numeral). This connection of the large-diameter portion and the small-diameter portion can be easily formed through a molten resin injection molding procedure (integral molding) without using an adhesive-bonding, etc.

The large-diameter portion 3.1LD has in its interior at the proximal end PE side an undercut portion 3.1UC (see Fig. 9(B)) in the shape of an annular stepped portion as will be detailed later and has in its interior at the distal end DE side the large-diameter portion fitting or mounting portion 3.1LDSN.

### (Small-diameter Portion 3.1SD)

The small-diameter portion 3.1SD has a so-called "substantially tubular" shape.

The small-diameter portion 3.1SD has on its exterior at the proximal end PE side a small-diameter portion fitting portion 3.1SDSN and, at the proximal end PE side, is fitted with the fin portion 3.1FN.

### (Fin Portion 3.1FN)

The fin portion 3.1FN of the small-diameter portion 3.1SD is configured from a plurality of fins 3.1FF and a fin flange 3.1FNF.

In other words, the fin portion 3.1FN has the plurality of fins 3.1FF (three fins in the exemplification of Fig. 9) and, at the proximal end PE side of the fins 3.1FF, is formed with the fin flange 3.1FNF as depicted in Fig. 9.

Spaces (or called "interspaces" and dare not be designated by reference letter and numeral) are disposed between each of the fins 3.1FF so that when subjected to an external clamping pressure from tightening of a nut 3.2 described later, each of the fins 3.1FF closes toward a substantial center (dare not be designated by reference letter and numeral) so as to fill up (infill) each of the spaces, thereby enabling the dilator 5 to be press-held and firmly fixed.

The above-described lock cap 3.1 has at its substantial center (dare not designated by reference letter and numeral) a through-hole 3.10 extending from the proximal end PE side of the small-diameter portion 3.1SD to the distal end DE side of the small-diameter portion 3.1SD (or large-diameter portion 3.1LD), and the dilator 5 is inserted into the through-hole 3.10.

The lock cap 3.1 is preferably made of a hard or rigid material, e.g., polypropylene resin, etc.

### [Nut 3.2]

As depicted in Fig. 3, the nut 3.2 is a member that is coupled to the lock cap 3.1 via their respective fitting portions such as screws (e.g. , double-threaded screws) having a following fixing function: in which the nut 3.2 and lock cap 3.1 are screwed together while the exterior of the lock cap 3.1 having the dilator 5 inserted therein is held by the nut 3.2, and by clamming the screw tight the dilator 5 is fixed immovably at the proximal end PE side of the hub 2. More specifically, the small-diameter portion fitting portion 3.1SDSN has a threaded portion, while the nut 3.2 has a nut portion in the form of an inner-tube portion fitting portion 3.2ISN, so that by rotating the two portions in relatively opposite directions the threaded portion and the nut portion in mesh become screwed tight together.

This point will hereinafter be described in more detail.

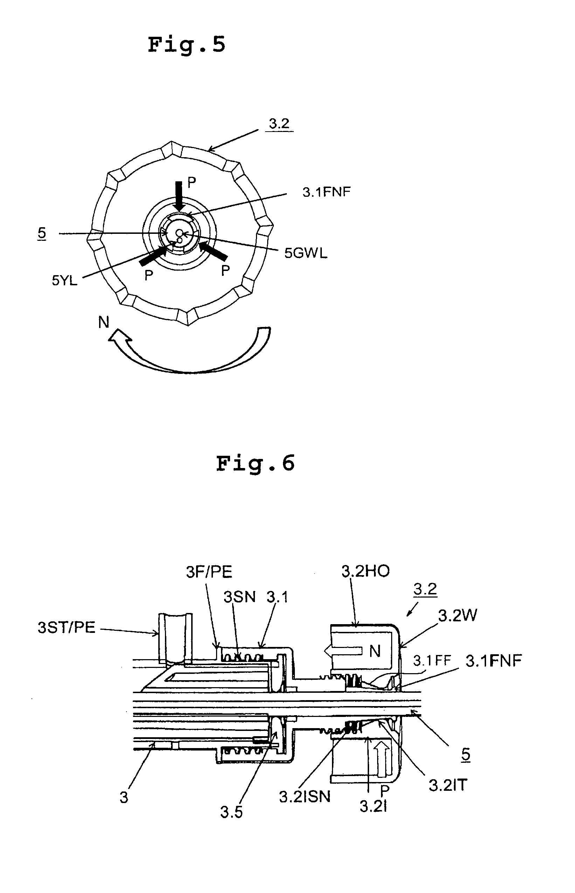

The plurality of fins 3.1FF of the lock cap 3.1 close toward the substantial center (dare not be designated by reference letter and numeral), to consequently fix the dilator 5 as depicted in Figs. 3 and 6.

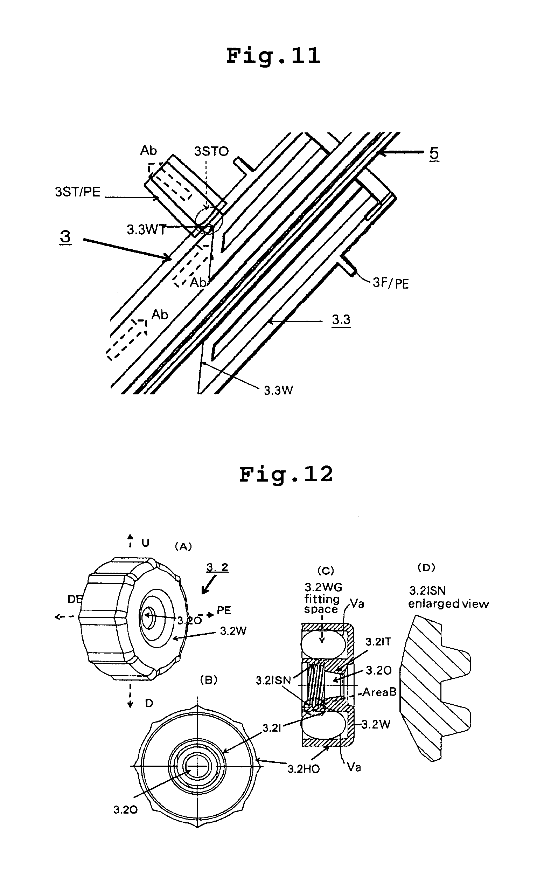

As depicted in Figs. 6 and 12 , the nut 3.2 has schematically an outer housing portion 3.2HO and an inner tube portion 3.2I.

The outer housing portion 3.2HO is a part that defines the contours of the nut 3.2 and has a so-called "substantially cylindrical" shape.

The inner-tube portion 3.2I is a part that defines the internal structure of the nut 3.2 and has a so-called "substantially tubular" shape.

The outer housing portion 3.2HO has a wall 3.2W continuously connected to it at its proximal PE side.

The wall 3.2W has at its substantial center (dare not be designated by reference letter and numeral) the inner-tube portion 3.2I protruding toward the distal end DE side.

The inner-tube portion 3.2I has in its interior at the distal end DE side the inner-tube portion fitting or mounting portion 3.2ISN.

The inner-tube portion 3.2I has in its interior at the proximal end PE side an inner-tube portion projecting portion 3.2IT.

The inner-tube portion projecting portion 3.2IT is shaped into a taper descending from the proximal end PE side toward the distal end DE side.

As depicted in Fig. 6, for example, when tightening the nut 3.2 by displacing and rotating the nut 3.2 toward the distal end DE direction (N-direction), the fins 3.1FF of the lock cap 3.1 are subjected to an external pressure (P-direction) from the tightening inner-tube portion projecting portion 3.2IT, with the result that the fins 3.1FF is forced to close toward the substantial center (dare not be designated by reference letter and numeral) thereby the dilator 5 is fixed. Figs. 4 and 5 depict the state where the dilator becomes fixed with the thus tightened nut 3.2, which clamped situation can be visibly recognized from outside by the fin flange 3.1FNF pressing against the outer surface of the dilator as shown in Fig.5.

The inner-tube portion 3.2I has at its substantial center (dare not be designated by reference letter and numeral) an insertion opening 3.20 extending from the proximal end PE side (through) to the distal end DE side, as exemplified in Fig. 12. The insertion opening 3.20 receives the dilator 5 as depicted in Fig. 6.

The nut 3.2 is preferably made of a hard resin material, e.g., a polypropylene resin, etc.

### [Nut Weight 3.2WG]

The nut weight 3.2WG is a member fitted or mounted to the nut 3.2 to increase the weight of the nut, to thereby steadily hold and fix the dilator. As exemplified in Fig. 15, the nut weight 3.2WG has a so-called "substantially tubular" shape and is fitted or mounted, from the distal end DE direction, into a space (referred to as a "nut weight fitting space" Va) (see Fig. 12(C)) between the outer housing portion 3.2HO and the inner-tube portion 3.2I of the nut 3.2.

The resulting nut 3.2 with an increased weight, enables the dilator 5 to be held and fixed more steadily.

As depicted in Fig. 15, the nut weight 3.2WG has at its substantial center (dare not be designated by reference letter and numeral) a nut weight through-hole 3.2WGO. The through-hole 3.2WGO receives the dilator 5 through it.

The nut weight 3.2WG is preferably made of a hard resin material having a large specific gravity, e.g., a polyacetal resin, polyurethane resin, etc.

### [Rectifying Member 3.3]

A rectifying member 3.3 is a member having a function of directing air generated in the sheath 2 and the sheath hub 3 at the time of priming or remaining air therein, toward the proximal-end-side hub side-tube 3ST/PE, thereby discharging it outwardly.

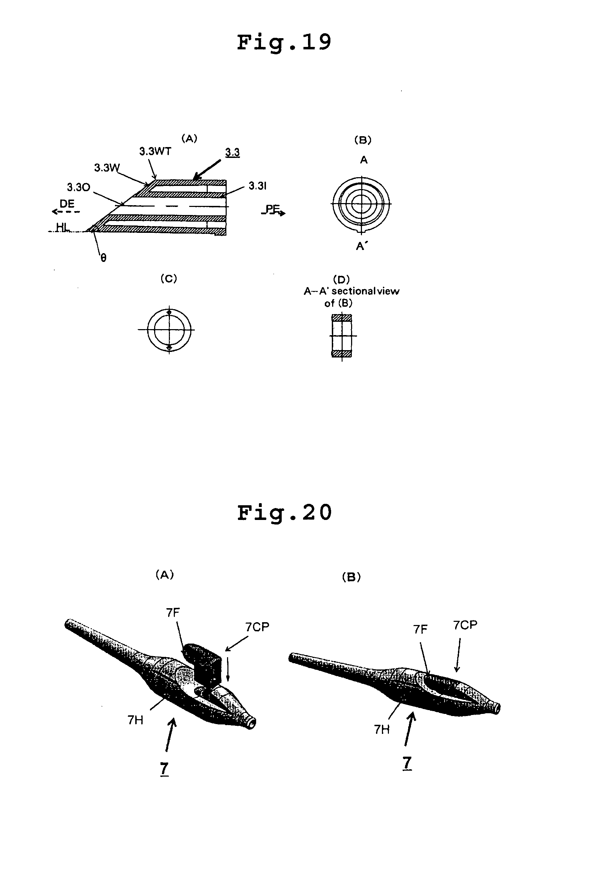

As exemplified in Fig. 19, the rectifying member 3.3 has a so-called "substantially tubular" shape and, as depicted in Fig. 3, is externally-dimensioned in shape to allow it to be fitted into, and disposed in the sheath hub 3 (more precisely, the hub base body 3BD). The rectifying member 3.3 has at its distal end DE side an inclined wall 3.3W (inclined upward from the distal end DE side toward the proximal end PE side) for guiding and inducing a smooth discharge of air.

The rectifying member 3.3 has a through-hole 3.30 at substantially center (dare not be designated by reference letter and numeral) as depicted in Fig. 19.

The through-hole 3.30 is an opening allowing the dilator 5 to be inserted therethrough.

The inclined wall 3.3W is one of the most characteristic parts in the rectifying member 3.3 and has a function of causing or allowing air displaced by the priming liquid Fl injected from the distal-end-side hub side-tube 3ST/DE at the time of priming and air Ab generated in the distal end DE-side interior of the sheath 2 to rise along the inclined wall 3.3W, thus leading the air into the proximal-end-side hub side-tube 3ST/PE for discharge outwardly, as exemplified in Figs. 10 and 11.

The rectifying member 3.3 is disposed such that a top portion 3.3WT of the inclined wall 3.3W is positioned at or near an opening 3STO (outlet) of the proximal-end-side hub side-tube 3ST/PE of the hub 3, as exemplified in Fig. 3. The reason is as follows.

When priming the stent graft indwelling device 1, it is performed while holding the device in an inclined position (to the left: to the bottom D side in the distal end DE direction) as exemplified in Fig. 10.

It is therefore effective to form the wall of the rectifying member 3.3 at the distal end DE as the inclined wall 3.3W that stands, when priming is performed, substantially at right angles or approximately at right angles, as depicted in Fig 11. As a result, air Ab ejected in priming rises from below to impinge on the inclined wall 3.3W and further moves straight upward along the substantially upright wall, to be discharged through the opening 3STO outward at the uppermost top part of the wall 3.3W.

Defining an angle *θ* of inclination of the inclined wall 3.3W as an angle at which an inclined line and a horizontal axis HL intersect at an acute angle as depicted in Fig. 19, the *θ* is in the range of 30° to 60°, preferably 40° to 50°, more preferably 43° to 47°, most preferably is approximately 45°.

Although in the exemplification of Fig. 19 the rectifying member 3.3 has in its interior a space (dare not be designated by reference letter and numeral) via which space the inner tube 3.3I is formed at substantially the center, the rectifying member 3.3 may be solid (in that case, only the through-hole 3.30 remains left, but the space and the inner tube 3.3I are not definitely defined nor formed).

The rectifying member 3.3 is made of, e.g., polypropylene resin, etc.

### [Hemostatic Valve 3.5 and Undercut Portion 3.1UC]

The hemostatic valve 3.5 is a sort of packing that is fitted or attached to an undercut portion 3.1UC of the lock cap 3.1 as exemplified in Figs. 3 and 6 to 9 to provide a pressure resistance capability. In this case, the "undercut portion 3.1UC" is an annular stepped portion projecting annularly from the inner wall portion of the wall 3.1LDW of the lock cap large-diameter portion as depicted in Fig. 9(B).

The hemostatic valve 3.5 has a so-called "substantially planar plate" shape as exemplified in Fig. 18. That is, it has the shape of a disc having an opening at its center and having a stepped flange 3.5F along its outer periphery as shown in Fig. 18.

More precisely, the hemostatic valve 3.5 has the flange 3.5 F formed in a stepped manner at the proximal end PE side. The flange 3.5F is fitted or attached to the stepped portion of the annularly stepped undercut portion 3.1UC of the lock cap 3.1.

The hemostatic valve 3.5 has the opening (insertion opening) 3.50 substantially at the center (dare not be designated by reference letter and numeral).

The insertion opening 3.50 is an orifice for allowing the dilator 5 to be inserted therethrough.

The hemostatic valve 3.5 is preferably made of a soft or resilient material such as rubber, silicone resin, etc.

### [Dilator 5]

The dilator 5 itself usable in the present invention is basically one having such a structure as described in e.g.,

Patent Document 1, etc., proposed by the present inventors and is formed from e.g. , an SUS pipe (rod or bar) or a material having a rigidity such as a hard resin.

As depicted in Fig. 1 of Cited Document 2 for example, the dilator 5 is, at its distal end DE side, mounted with the proximal end PE of the front tip 7 (dare not be depicted).

As depicted in Fig. 1, the dilator 5 is attached with the proximal end connector 8 (substantially Y-shaped branching tube) at its proximal end PE side.

As depicted in Figs. 5 and 8, etc. , from the proximal end PE side to the distal end DE side, the dilator 5 is formed or provides at its section with a wire lumen 5YL for allowing a wire (or also called yarn or filament) to pass therethrough, and also with a guide wire lumen 5GWL for allowing a guide wire GW to pass therethrough.

As described in Cited Document 1, etc. , the wire (dare not be depicted) is used to adjust the position of indwelling and the angle of insertion of the stent graft, wherein by adjusting the tension applied to the wire, the adjustment of the position of indwelling and the angle of insertion of the stent graft can be made.

### [Front tip 7]

The front tip 7 is a member that has at its top a hook 7F (see Fig. 20(B)) and that is mounted or attached to the dilator 5 at the distal end DE side, in which the stent graft to be indwelled is hooked to the hook 7F, and thereby adjustment of the position of indwelling and the insertion angle of the stent graft is made. As the front tip 7, one already disclosed in Cited Document 1, etc., is preferably applicable.

As exemplified in Fig. 20, the front tip 7 has a main body 7H and a cap 7CP. The cap and the main body are formed separably as depicted in Fig. 20(A) and, when in use, the cap is fitted and integrated into the main body for use as depicted in Fig. 20(B).

The front tip 7 is integrally formed in a streamline shape as shown in Fig. 20, which thereby may not inflict injury to the interior of the blood vessel when in operation.

Since in this manner, the front tip 7 is separably formed into the main body and the cap (two-portion tip), the direction of the front tip 7 in a blood vessel can be visually and securely recognized from outside, by varying the concentration (added amounts) of a contrast medium, e.g., barium sulfate mixed into the main body 7H and the cap 7CP (e.g., adding 40% of BASO₄ to the main body 7H and 10% of BASO₄ to the cap 7CP), and by allowing X-ray images of the main body and the cap to be displayed on a monitor, thus producing clearly-discernable different gray-scale images for each part of the tip.

As exemplified in Fig. 21, by covering the portion of the hook 7F of the front tip 7 with the sheath 2 at the distal end DE side (distal-end-side sheath 2DE), the blood vessel can be prevented from being injured due to a direct contact of the hook 7F with the blood vessel wall when approaching a diseased area.

The front tip 7 is made of a material having a proper hardness and flexibility among synthetic resins such as a polyamide-based elastomer, polyurethane, and polyvinyl chloride.

In the above description, as to the basic technical contents of the front tip 7, the sheath, the dilator, etc., the technical contents disclosed in Patent Documents 1 and 2 that are the prior applications of the present inventors can be herein incorporated by reference and the descriptions of the prior applications can be referred to for the parts not detailed in the description of the present invention.

### (Assembling of Stent Graft Indwelling Device 1)

Description will be given of an example of a method for assembling members comprising the sheath 2, the hub 3, the dilator 5, the lock cap 3.1 and nut 3.2 acting as fixing members, the rectifying member 3.3, and the hemostatic valve 3.5 of the stent graft indwelling device 1 of the present invention described hereinabove.

### (Preparation Stage)

(1) The hemostatic valve 3.5 having a shape as depicted in Fig. 18 for example is mounted to the lock cap 3.1. Specifically, the hemostatic valve 3.5 is attached to the undercut portion 3.1UC of the lock cap 3.1, with the flange 3.5F of the hemostatic valve 3.5 being fitted to the annular stepped portion of the undercut portion 3.1UC.

### (First State)

(2) To the hub 3 at the proximal end PE side, the large-diameter portion 3.1LD at the distal end DE side of the lock cap 3.1 is fitted. This is a "first state" (see Fig. 7).

### (Start State of Transition and Transition (Intermediate) State)

(3) The lock cap 3.1 is tightened toward the distal end DE direction as indicated by an arrow I of Fig. 8 (this is a "start state of transition") . The proximal-end PE-side end of the hub 3 comes into contact with the distal-end DE-side end of the hemostatic valve 3.5, thus resulting that the exterior of the hemostatic valve 3.5 is gradually getting compressed between the proximal-end PE-side end of the hub 3 and (the inner wall of) the large-diameter portion wall 3.1LDW of the lock cap 3.1 (this is a "transition (or intermediate) state").(In Fig. 8, an area C enclosed by a broken line indicates the area (region) compressed by the arrow I.)

### (Final State of Transition) (Second State)

(4) The wall of the interior of the hemostatic valve 3.5 (in other words, the exterior of the insertion opening 3.5O), being compressed as in (3), having no way to escape but to deform toward the center (dare not be designated by reference letter and numeral), i.e. , toward the dilator 5 as indicated by a triangular arrow II of Fig. 8, increasing the area of contact with the exterior of the dilator 5. This is a "final state of transition" . In this state, the interior of the hemostatic valve 3.5 applies an external pressure to the exterior of the dilator 5, thereby achieving a strong intimate fixing therebetween. This corresponds also to a "second state".

In the present invention, the device in this final state with remarkably-improved pressure resistance performance or capability, is finally and stably achieved, so that when the indwelling device is inserted into an artery, the backflow of arterial blood into the sheath or the dilator can be effectively prevented.

### (Fixing of Dilator)

(5) Furthermore, as exemplified in Figs. 5 and 6, the nut 3.2 is rotationally displaced toward the distal end DE direction (the proximal end PE direction of the lock cap 3.1) with the clockwise rotation (see an arrow N of Figs. 5 and 6).

The lock cap 3.1 and the nut 3.2 are coupled via a screw to each other and are screwed together by tightening the screw, to consequently hold and fix the dilator 5.

Furthermore, by external tightening accompanied by displacement of the nut 3.2, the three fins 3.1FF close toward the center so that the dilator 5 can be firmly and immovably fixed (see arrows P of Figs. 5 and 6).

### (Priming and Air Bleeding of Stent Graft Indwelling Device 1)

An example of priming and air bleeding of the stent graft indwelling device 1 will then be described.
(1) Tilting the stent graft indwelling device 1 approximately 45° to the left, as exemplified in Figs. 10 and 11, priming liquid Fl is introduced from the distal-end-side hub side-tube 3ST/DE into the sheath 2.
(2) Air Ab accumulated in the sheath 2 at the distal end DE side moves upward from the distal end DE side toward the proximal end PE side. Air Ab is guided by the inclined wall 3.3W of the rectifying member 3.3, so that it can promptly be discharged to the outside through the top portion 3.3WT and then the opening 3STO of the proximal-end-side hub side-tube 3ST/PE.

### INDUSTRIAL APPLICABILITY

In the stent graft indwelling device of the present invention, the lock cap 3.1 and the hemostatic valve 3.5 are arranged together, which, in combination, enables a remarkably-improved pressure resistance performance of the sheath hub; further, in the present invention, the lock cap 3.1 and the nut 3.2 are also arranged together, which, in combination, enables a remarkably-improved fixing performance of the dilator 5; and furthermore, in the present invention, the introduction of the rectifying member 3.3 establishes a remarkably-improved air bleeding capability while priming procedure of the sheath 2 and the sheath hub 3 is conducted, these advantages of the present invention thus provide extremely great industrial applicability in the medical field.

### EXPLANATIONS OF LETTERS AND NUMERALS

1 stent graft indwelling device
2 sheath
3 sheath hub
3BD hub base body
3ST hub side-tube
3ST/DE distal-end-side hub side-tube
3ST/PE proximal-end-side hub side-tube
3SN hub fitting or mounting portion
3STO opening (outlet) of 3ST/PE
3TP tapered portion
3HT thin or small tube portion
3F hub flange
3F/PE proximal-end-side hub flange
3F/DE distal-end-side hub flange
3SR strain relief (antikink cover)
3SRM fit-in groove
3SRDS stepped portion
SRTP taper portion
3GP hub grip
3GPCT connection tube
3GPCTK uneven portion
3GPS connecting member
3.1 lock cap
2.1LD large-diameter portion
3.1LDSN large-diameter portion fitting or mounting portion
3.1LDW large-diameter portion wall
3.1SD small-diameter portion
3.1SDSN small-diameter portion fitting or mounting portion
3.1UC undercut portion
3.1FN fin portion
3.1FF fin
3.1FNF fin flange
3.10 through-hole
3.2 nut
3.2HO outer housing portion
3.2I inner-tube portion
3.2ISN inner-tube portion fitting or mounting portion
3.2IT inner-tube portion projecting portion
3.2W wall
3.20 insertion opening
3.2WG nut weight
3.2WGO nut weight through-hole
3.3 rectifying member
3.3W rectifying-member inclined wall
3.3WT top portion
3.30 through-hole
3.3I inner tube
3.5 hemostatic valve
3.50 insertion opening
3.5F flange
5 dilator
5YL wire lumen
5GWL guide wire lumen
7 front tip
7H main body
7CP cap
7F hook
8 proximal end connector
Ab air
Fl priming liquid
Va nut weight fitting space

## Claims

1. A stent graft indwelling device (1) comprising a sheath (2), a sheath hub (3), a dilator (5), and a front tip (7),
the front tip (7), the sheath (2), and the sheath hub (3) being disposed from a distal end DE toward a proximal end PE in this order,
the dilator (5) being inserted from the proximal end PE side of the sheath hub (3) toward the distal end DE side of the sheath (2),
a lock cap (3.1) and a hemostatic valve (3.5) being arranged as pressure resistant members at the proximal end PE side of the sheath hub (3),
the lock cap (3.1) having a large-diameter portion (3.1LD), a small-diameter portion (3.1SD), and a fin portion (3.1FN),
the large-diameter portion (3.1LD) having at the proximal end PE side a large-diameter portion wall (3.1LDW),
the large-diameter portion (3.1LD) having on its interior at the proximal end PE side an undercut portion (3.1UC) comprised of an annular step portion,
the hemostatic valve (3.5) having generally a form of a planar plate, and having at the proximal end PE side a flange (3.5F) and having at its substantially center an insertion opening (3.50), wherein
the flange (3.5F) of the hemostatic valve (3.5) is mounted to the annular step portion of the undercut portion (3.1UC) of the lock cap (3.1),
thereby fixing the hemostatic valve (3.5) to the undercut portion (3.1UC),
the large-diameter portion (3.1LD) having a large-diameter portion fitting portion (3.1LDSN) formed on its interior at the distal end DE side,
the distal end DE side of the small-diameter portion (3.1SD) being connected to a substantial center of the large-diameter portion wall (3.1LDW),
the small-diameter portion (3.1SD) having a small-diameter portion fitting portion (3.1SDSN) formed on its exterior at the proximal end PE side,
the small-diameter portion (3.1SD) being fitted, at the proximal end PE side, with the fin portion (3.1FN),
the fin portion (3.1FN) having a plurality of fins (3.1FF),
the large-diameter portion (3.1LD) at the distal DE side of the lock cap (3.1) being fitted to the proximal end PE side of the sheath hub (3).

2. The stent graft indwelling device (1) as recited in claim 1, wherein
the lock cap (3.1) defined in claim 1 and a nut (3.2) are arranged at the proximal end PE side of the sheath hub (3), as fixing members for the dilator (5),
the nut (3.2) having an outer housing portion (3.2HO) and an inner-tube portion (3.2I),
the outer housing portion (3.2HO) having at the proximal end PE side a wall (3.2W), and the inner-tube portion (3.2I) projecting from a substantial center of the wall (3.2W) toward the distal end DE side,
the inner-tube portion (3.2I) having an inner-tube portion fitting portion (3.2ISN) formed on its interior at the distal end DE side,
the inner-tube portion (3.2I) having an inner-tube portion projecting portion (3.2IT) formed on its interior at the proximal end PE side,
the nut (3.2) having an insertion opening (3.20) substantially at its center, and wherein
the nut (3.2) is rotationally displaced toward the distal end DE direction, thereby
external tightening accompanied by the displacement of the nut (3.2) causes the plurality of fins (3.1FF) of the lock cap (3.1) to close toward the center so that the dilator (5) can be fixed immovably.

3. A stent graft indwelling device (1) comprising a sheath (2), a sheath hub (3), a dilator (5), and a front tip (7),
the front tip (7), the sheath (2), and the sheath hub (3) being disposed from a distal end DE toward a proximal end PE in this order,
the dilator (5) being inserted from the proximal end PE side of the sheath hub (3) toward the distal end DE side of the sheath (2),
the sheath hub (3) having on its exterior a distal-end-side hub side-tube (3ST/DE) and a proximal-end-side hub side-tube (3ST/PE),
a rectifying member (3.3) being disposed as an air bleeding member on the interior at the proximal end PE side of the sheath hub (3),
the rectifying member (3.3) having a substantially tubular shape and having at the distal end DE side an inclined wall (3.3W),
a top portion (3.3WT) of the inclined wall (3.3W) being positioned in the vicinity of an opening (3STO) of the proximal-end-side hub side-tube (3ST/PE), wherein
air generated in a priming liquid introduced from the distal-end-side hub side-tube (3ST/DE) into the sheath hub (3) and into the sheath (2) can be discharged outwardly through a passage or by way of the inclined wall (3.3W) of the rectifying member (3.3), the top portion (3.3WT), and the opening (3STO) of the proximal-end-side hub side-tube (3ST/PE).

4. The stent graft indwelling device (1) as recited in claim 3, wherein
an angle of the inclined wall (3.3W) of the rectifying member (3.3) is formed in the range of 30° to 60°.

5. The stent graft indwelling device (1) as recited in any one of claims 1 to 4, wherein
the exterior of a connection between the proximal end PE side of the sheath (2) and the distal end DE side of the sheath hub (3) is covered with a strain relief (3SR),
the strain relief (3SR) having a substantially tubular shape and being formed, with a fit-in groove (3SRM) on its interior at the proximal end PE side,
a distal-end-side hub flange (3F/DE) of the sheath hub (3) being fitted in the fit-in groove (3SRM).

6. The stent graft indwelling device (1) as recited in any one of claims 1 to 5, wherein
the exterior of the sheath hub (3) is covered with a hub grip (3GP),
the hub grip (3GP) having along its longitudinal L direction a plurality of connection tubes (3GPCT) and a plurality of connecting members (3GPS),
the plurality of connection tubes (3GPCT) being connected to each other so as to form a gap therebetween or apart from each other with a space therebetween,
the connection tube (3GPCT) being formed, on its exterior, with an uneven portion (3GPCTK).
